## Europäisches Patentamt

## European Patent Office

## Office européen des brevets

(11) Veröffentlichungsnummer: **0 140 061**
**B1**

(12) # EUROPÄISCHE PATENTSCHRIFT

(45) Veröffentlichungstag der Patentschrift:
**19.04.89**

(21) Anmeldenummer: **84110663.6**

(22) Anmeldetag: **07.09.84**

(51) Int. Cl.⁴: **A 23 F 3/36,** B 01 D 11/02,
C 07 D 473/12, A 23 F 5/20

(54) **Verfahren und Vorrichtung zur Extraktion von Inhaltsstoffen aus Naturprodukten.**

(30) Priorität: **28.10.83 DE 3339181**

(43) Veröffentlichungstag der Anmeldung:
**08.05.85 Patentblatt 85/19**

(45) Bekanntmachung des Hinweises auf die Patenterteilung:
**19.04.89 Patentblatt 89/16**

(84) Benannte Vertragsstaaten:
**AT CH DE FR GB IT LI NL**

(56) Entgegenhaltungen:
**EP-A-0 159 724**
**FR-A-2 360 261**
**FR-A-2 361 824**
**FR-A-2 394 547**

(73) Patentinhaber: **Uhde GmbH, Friedrich- Uhde-Strasse 15 Postfach 262, D-4600 Dortmund 1 (DE)**

(72) Erfinder: **Theissing, Peter, Dr., Fuchsweg 26, D-4600 Dortmund 30 (DE)**
Erfinder: **Körner, Jörg- Peter, Dipl.- Ing., Baurothstrasse 7, D-5800 Hagen 1 (DE)**
Erfinder: **Saamer, Peter, Dipl.- Ing., Letteweg 36, D-5860 Iserlohn- Hennen (DE)**

(74) Vertreter: **Patentanwälte Meinke und Dabringhaus Dipl.- Ing. J. Meinke Dipl.- Ing. W. Dabringhaus, Westenhellweg 67, D-4600 Dortmund 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

### Beschreibung

Die Erfindung richtet sich auf ein Verfahren und eine Vorrichtung zur Extraktion von Inhaltsstoffen aus Naturprodukten mittels eines unter Druck stehenden Fluids, vorzugsweise zur Entkoffeinierung von Teeblätters mittels $CO_2$, wobei anschließend die Inhaltsstoffe an einen Adsorbenten, vorzugsweise Aktivkohle, gebunden werden.

Es ist bekannt, z. B. Teeblätter in einem Hochdruckbehälter einzubringen, der axial von unter Druck stehendem $CO_2$ durchströmt wird. Dieses $CO_2$ wird anschließend in einem seperaten Adsorptionsbehälter geführt und das Koffein dort adsorbiert. Dabei ist es bekannt, daß $CO_2$ im Kreislauf zu führen.

Die bekannte Verfahrensweise hat ganz erhebliche Nachteile. Diese bestehen u. a. darin, daß aus Festigkeitsgründen sehr schlanke, lange Behälter gebaut werden müssen, was zu sehr großen Schütthöhen an Teeblättern führt. Dies führt wiederum zu sehr langen Extraktionszeiten, weil die $CO_2$-Strömungsgeschwindigkeit niedrig sein muß. Erkennbar werden die dabei in Strömungsrichtung bergab zuerst liegenden Teeblätter permanent mit $CO_2$ beaufschlagt und immer noch extrahiert, wenn das dort vorhandene Koffein bereits längst ausgezogen ist. Das Verfahren muß nämlich so lange angewandt werden, bis auch die letzt aufliegende Teeblattschicht behandelt wurde. Bis dies geschehen ist, entzieht das $CO_2$ den zuvor beaufschlagten Teeblattschichten auch das Aroma.

Ein weiterer Nachteil besteht darin, daß aufgrund hoher Strömungsgeschwindigkeiten die Teeblattschichten die Neigung haben, zu verbacken. Auch führt die Bauweise dazu, daß ein Teil der im Fluid gelösten Inhaltsstoffe an Behälterwandungen sowie in Rohrleitungen und Ventilen ausfällt und zu Verstopfungen führt. Ein weiterer Nachteil besteht darin, daß bei konstanter Strömungsgeschwindigkeit innerhalb der Teeblattschicht die Entkoffeinierungsgeschwindigkeit immer stärker abnimmt, je stärker das $CO_2$ mit Koffein beladen ist.

Aufgabe der Erfindung ist die Schaffung einer Lösung, mit der die notwendige Behandlungszeit der zu behandelnden Naturprodukte verringert wird, mit der die vom beladenen Fluid durchströmten wege verkürzt und damit die Gefahr von Verunreinigungen verringert und mit der insbesondere die insgesamt aufgeprägte Fluidströmungsgeschwindigkeit verringert wird.

Mit einem Verfahren der eingangs bezeichneten Art wird diese Aufgabe gemäß der Erfindung dadurch gelöst, daß eine Schichtdicke des Naturproduktes vorgesehen wird, die klein im Verhältnis zur Schichtabmessung quer zur Strömungsrichtung ist, und das Fluid durch die Schicht des Naturproduktes mit sich in Strömungsrichtung ändernder, insbesondere steigender Geschwindigkeit geführt wird.

Mit der Erfindung wird vorteilhaft erreicht, daß das Naturprodukt in kleinerer Schichtdicke vorliegen kann, so daß der Druckverlust geringer ist. Gleichzeitig wird eine Verpressung des Naturproduktes in Strömungsrichtung des Fluides vermieden. Eine gleichmäßige Durchströmung des Naturproduktes läßt sich erreichen, ebenso wie erheblich geringere Anströmgeschwindigkeiten. Bei Hintereinanderschaltung mehrerer Naturstoff/Adsorbentenschichten wird die Umwälzmenge des Fluids verringert, womit alle damit verbundenen Anlagenteile kleiner und damit kostengünstiger ausgeführt werden können.

Ein weiterer Vorteil liegt darin, daß der bei gleichbleibender Fluidgeschwindigkeit zu erwartende Abfall des Extraktionsgrades in Strömungsrichtung dadurch vermieden wird, daß bei steigender Geschwindigkeit der Stoffübergangs- und damit der Entkoffeinierungskoeffizient steigt, was zur vergleichsmäßigung der Extraktion innerhalb z. B. der Teeschicht führt.

In Ausgestaltung sieht die Erfindung vor, daß das beladene Fluid ohne Zeitverzug unmittelbar nach Verlassen der Naturproduktschicht dem Adsorbenten zugeführt wird. Damit lassen sich insbesondere eine Verschmutzung des Gases und dadurch bedingte Ablagerungen in Apparaten und Rohrleitungen verhindern. Das im Kreis geführte Fluid steht dann jeweils wieder beladungsfrei und sauber zur Verfügung.

Vorteilhaft ist es, wenn das Fluid beim Anströmen der Schicht des Naturproduktes eine große Durchströmfläche beim Verlassen der Naturproduktschicht eine demgegenüber wesentlich kleinere Abströmfläche durchströmt. Diese Verfahrensweise läßt vergleichsweise einfache konstruktive Verwirklichungen zu, entweder durch sich in Stromabwärtsrichtung verjüngende Druckgefäßgehäuse oder durch Konstruktionen, die ein Strömen von einer zylindrischen Außenwand in ein Zylinderinneres ermöglichen, womit zwangsläufig eine Durchtrittsflächenverringerung für das strömende Fluid verbunden ist.

Nach der Erfindung kann auch vorgesehen sein, daß das Fluid in wechselnder Folge eine Naturproduktschicht, den Adsorbenten, erneut eine Naturproduktschicht und wieder einen Adsorbenten durchströmt, und so fort, wobei dies in unterschiedlichen Anlageteilen hintereinandergeschaltet ebenso vorgesehen sein kann, wie in einem einzigen Anlageteil, nur in unterschiedlichen Packungsschichten.

Die eingangs genannte Aufgabe wird mit einer Vorrichtung zur Durchführung des Verfahrens dadurch gelöst, daß die Vorrichtung als im wesentichen zylindrischer Hochdruckbehälter mit im wesentlichen kreiszylinderförmigen Einsätzen zur Aufnahme des Naturproduktes und/oder des Adsorbenten ausgebildet und die Gasführung von einem äußeren Bereich des Zylinders in das Innere des Zylinders vorgesehen ist. Diese Vorrichtung ist konstruktiv einfach, sie ist insbesondere für hohe Drücke besonders gut geeignet und bietet eine sichere und zuverlässige Führung des behandelnden Fluids.

In Ausgestaltung kann vorgesehen sein, daß der zylindrische Hochdruckbehälter mit einem gasdichten Boden und einem gasdichtem Deckel ausgerustet ist und der außere Einsatz zur Aufnahme des Naturproduktes einen geringfügigen Abstand zur Innenfläche der zylindrischen Außenwand zur Bildung eines zylindrischen Abströmfläche für das Fluid aufweist, wobei der Fluidabzug nach Durchströmen des Naturproduktes sowie eines Adsorbenten am zentrischen zylindrischen Innenraum der Vorrichtung vorgesehen ist.

Mit dieser Ausgestaltung läßt sich eine sehr kompakte Bauweise der Vorrichtung erreichen. Sie ist leicht zu beschicken und das behandelte Produkt kann ebenso leicht entnommen werden, wie ggf. der Adsorbent ausgetauscht werden kann.

In weiterer Ausgestaltung kann nach der Erfindung eine Mehrzahl von Einsätzen zur Aufnahme des Naturproduktes und des Adsorbenten konzentrisch zueinander in der Vorrichtung vorgesehen sein, wobei es besonders zweckmäßig ist, wenn die Folge von zylindrischen Einsätzen mit Naturprodukten und dem Adsorbenten abwechselnd vorgesehen ist.

Um eine möglichst gleichmäßige Anströmung und Durchströmung des Gases sowohl durch das zu behandelnde Naturprodukt als auch durch den oder die Adsorbenten zu gewährleisten, sieht die Erfindung auch vor, daß die jeweils außen liegende Anströmwand für das Fluid mit Einrichtungen, wie Schikanen, Lochungen o. dgl. zur gleichmäßigen Verteilung des das Naturprodukt anströmenden Gases ausgerüstet sind.

Diese beim Anströmen zweckmäßige Maßnahme wird beim Abströmen aus dem Naturprodukt durch die unmittelbar dahinterliegende Adsorbtionsschicht erreicht, die auch zur vergleichmäßigung des Druckes innerhalb der Vorrichtung führt. Reicht dies nicht aus, können auch an den Trennflächen zwischen Naturprodukt und Adsorbtionsmittel entsprechende Einbauten, wie Löcher, Schikanen o. dgl. vorgesehen sein.

War oben vom Naturprodukt, insbesondere von Tee und Kaffee die Rede, so kann die hier beanspruchte Verfahrensweise und die beanspruchte Vorrichtung auch zur Behandlung anderer Naturprodukte herangezogen werden, ggf. ohne das Vorsehen eines Adsorbenten, dann, wenn das Extrakt direkt einer Weiterverarbeitung zugeführt werden soll. Um hier ein Beispiel zu nennen, sei der Entzug von Rosenöl genannt.

In einem abgewandelten Ausführungsbeispiel sieht die Erfindung auch vor, daß der Adsorbent in einem inneren Einsatzkorb und das Naturprodukt im Ringraum zwischen dem Adsorbenten und der Behälterwand eingebracht ist, wobei die Behälterwand wenigstens bereichsweie porös und vom Fluid durchströmbar ausgebildet ist. Diese Gestaltung bringt besondere Vorteile mit sich. So kann der Behälter so ausgestaltet sein, daß durch einen gemeinsamen Deckel, der dem Adsorbenten, z. B. der Aktivkohle, aufnehmende Käfig zentrisch eingebracht werden kann und gleichzeitig auch der Ringraum befüllbar ist, z. B. mit Kaffeebohnen o. dgl. Damit vereinfacht sich die konstruktive Gestaltung wesentlich.

Die Erfindung ist nachstehend anhand der Zeichnung beispielsweise näher erläutert. Diese zeigt in

Fig. 1   die linke Schnittdarstellung einer Hälfte einer Vorrichtung

Fig. 2   die rechte Hälfte einer Schnittdarstellung eines abgewandelten Ausführungsbeispieles der Vorrichtung nach der Erfindung sowie in

Fig. 3   die Gesamtdarstellung im Schnitt einer Vorrichtung nach einem weiteren Ausführungsbeispiel.

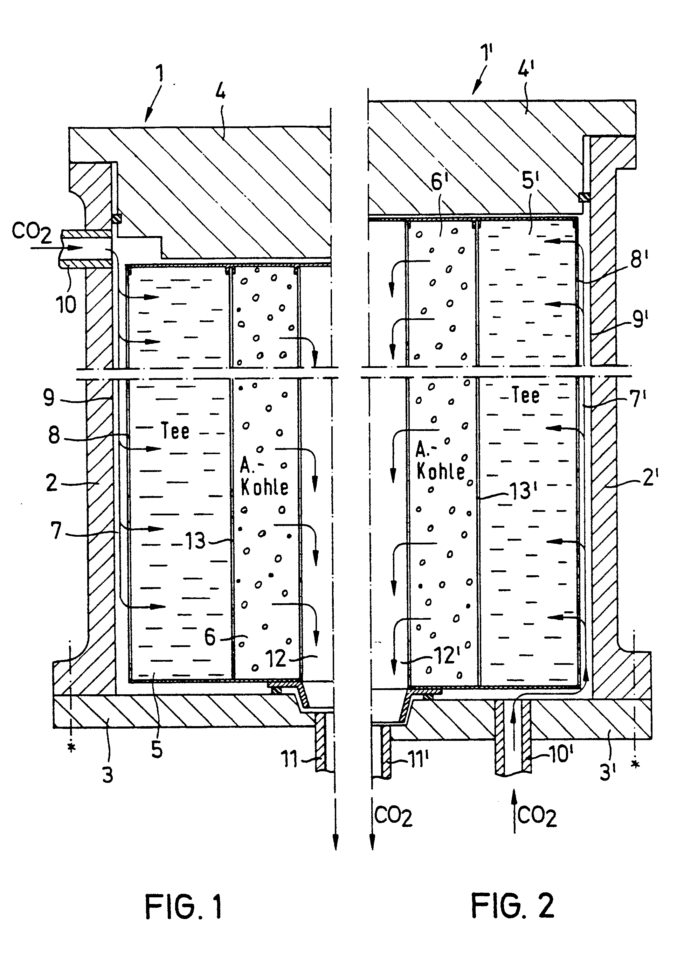

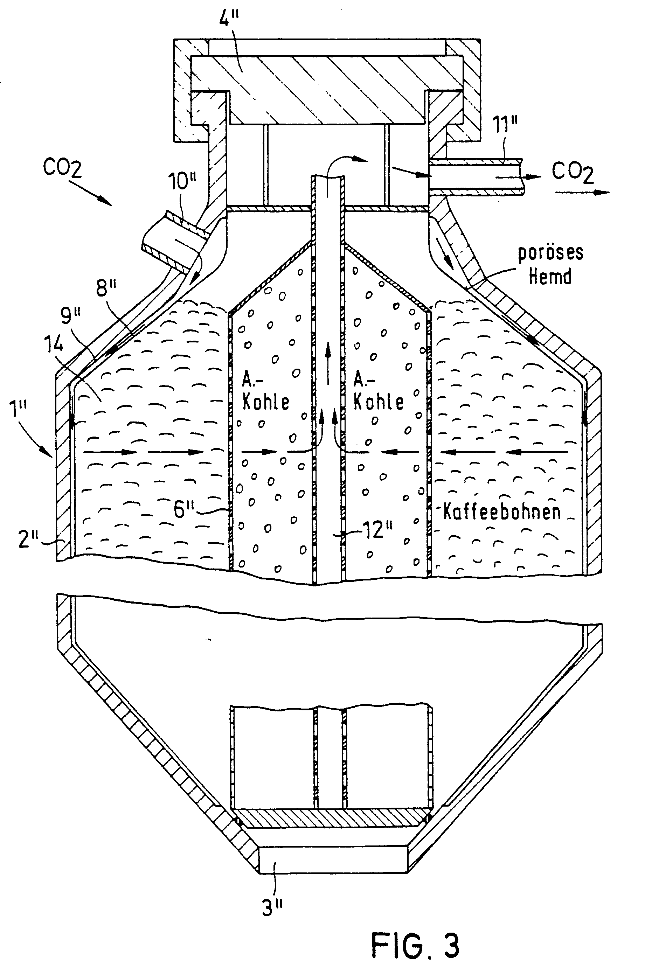

Die Vorrichtung als Hochdruckbehälter ist in den Figuren 1 und 2 in zwei unterschiedlichen Ausführungsvarianten dargestellt und in der Figur 1 mit 1, in der Fig. 2 mit 1' bezeichnet. Alle gegenüber der Fig. 1 wirkmäßig gleichen Bauteile und Ausbildungsformen sind, so weit wie möglich, in Fig. 2 mit den gleichen Bezugszeichen, allerdings gestrichen versehen. Dies gilt auch für das weitere Ausführungsbeispiel nach Fig. 3, hier sind die Bezugszeichen doppelt gestrichen.

Zunächst seien die Figuren 1 und 2 beschrieben:

Der zylindrische Hochdruckbehälter 1 ist mit einer Zylinderwand 2 und einem daran gasdicht angeordneten Boden 3 sowie Deckel 4 ausgerüstet. Es sei bemerkt, daß in der Praxis dieser zylindrische Hochdruckbehälter z. B. eine Längserstreckung von 3 m und mehr aufweisen kann.

Innerhalb des Hochdruckbehälters 1 sind kreiszylinderförmige Einsätze 5 für das blättrige Naturprodukt, z. B. Tee, und 6 für das Adsorbtionsmittel, z. B. Aktivkohle, eingesetzt, wobei ein Kreisringspalt 7 zwischen Außenzylinderfläche 8 des Einsatzes 5 für das Naturprodukt einerseits und der innenliegenden Zylinderwand 9 des Hochdruckbehälters andererseits freigelassen ist, um einen Durchströmraum für das zur Behandlung vorgesehene Fluid, z. B. $CO_2$, zu bilden.

Die Zuführöffnungen für das $CO_2$, mit 10 bzw. 10' bezeichnet, sind in den Abbildungsbeispielen in der Wand 2 bzw. im Boden 3 des Hochdruckbehälters vorgesehen. Sie können selbstverständlich auch im Deckel oder in der Zylinderwand vorgesehen sein. Allerdings ist die Boden- bzw. Wandkonstruktion deswegen günstiger, um nicht Versorgungsleitungen beim Öffnen des Deckels mitentfernen zu müssen.

Bei dem in Fig. 1 dargestellten Ausführungsbeispiel strömt das $CO_2$ seitlich in den Kreisringraum 7, durchströmt dann das Teepaket 5, anschließend das Adsorbtionspaket 6 und wird schließlich unten durch einen Entnahmestutzen 11 abgezogen und, im Kreislauf geführt, bei 10 unter Zwischenschaltung einer Förderpumpe (nicht dargestellt) wieder eingeführt.

Beim Ausführungsbeispiel nach Fig. 2 befindet sich sowohl der Zuführstutzen 10' als auch der Entnahmestutzen 11' für das $CO_2$ im Boden 3'.

Der innere Abströmraum des $CO_2$ ist in den Figurenhälften mit 12 bzw. 12' bezeichnet. Erkennbar steht dem strömenden Gas eine große Eintrittsfläche 8 in das Teepaket 5 zur Verfügung, während die Abströmfläche aus dem Teepaket, mit 13 bezeichnet, kleiner ist. Damit wird das $CO_2$ auf seinem Weg durch das Teepaket 5 beschleunigt, wodurch sich die weiter oben beschriebenen Vorteile ergeben.

In den Fig. 1 und 2 ist nicht näher dargestellt, daß sich sowohl das Naturprodukt, wie Tee, Rosenblätter o. dgl., in kreiszylinderförmigen Einsätzen, wie Körben o. dgl. untergebracht ist, die nach Entfernung des Deckels 4 nach oben aus der Vorrichtung 1 entnommen werden können. Ebenso ist in den Fig. 1 und 2 nicht dargestellt, daß eine Mehrzahl, vorzugsweise in abwechselnder Folge, von kreiszylinderförmigen Einsätzen zur Aufnahme des Adsorbenten andererseits innerhalb einer Vorrichtung vorgesehen sein kann. Natürlich kann auch eine Mehrzahl von Hochdruckbehältern 1 in Strömungsrichtung des zu behandelnden Fluids hintereinandergeschaltet oder auch parallel sein, wenn dies zweckmäßig sein sollte.

Beim Ausführungsbeispiel nach Figur 3 besteht der Hochdruckbehälter 1'' aus einem Zylinder 2'' mit sich oben und unten verjüngenden Bereichen, wobei der obere Bereich mit einem Deckel 4'' verschlossen ist, während der untere Bereich mit einer Öffnung 3'' versehen ist.

Die Zylinderwand 2'' ist auf ihrer Innenfläche 9'' mit einer porösen Beschichtung 8'' ausgerüstet, durch die z. B. $CO_2$ ins Innere des Behälters 1'' strömen kann.

Zentrisch in der Mitte befindet sich ein Aufnahmekorb 6'' für Aktivkohle, der solche Abmessungen hat, daß er nach Abnahme des Deckels 4'' aus der Vorrichtung entnommen werden kann. Der Bodenbereich dieses Einsatzes ist selbstdichtend ausgeführt, so daß der Behälter 1'' an seinem Boden 3'' nicht besonders zusätzlich verschlossen sein muß. Der Ringraum 14 zwischen der Zylinderwand 2'' und dem Einsatz 6'' für die Aktivkohle ist im dargestellten Beispiel mit Kaffebohnen gefüllt. Der $CO_2$ Zuführungsstutzen ist mit 10'' bezeichnet, während der Abführstutzen für das beladene $CO_2$ mit 11'' bezeichnet ist. Dieser Stutzen steht mit dem Abströmraum 12'' im Inneren des Käfiges 6'' in Verbindung, was durch die wiedergegebenen Strömungspfeile angedeutet ist.

Die Erfindung ist nicht auf die dargestellten Ausführungsbeispiele beschränkt. So kann beispielsweise innerhalb der Vorrichtung nur der Einsatz zur Aufnahme des Naturproduktes vorgesehen sein, während das Adsorptionsmittel in einem anderen Geräteteil außerhalb der Vorrichtung 1 untergebracht ist. Auch die zylindrische Bauweise ist nicht die einzige Möglichkeit den Erfindungsgedanken zu verwirklichen. So kann eine, in Strömungsrichtung des Fluids gesehen, stufenförmige Bauweise mit sich verringernden Querschnitten kastenförmiger Einsätze innerhalb eines Gehäuses vorgesehen sein u. dgl. mehr.

Zur Vergleichmäßigung und besseren Verteilung des behandelnden Fluids kann die Anströmwand 8 mit Einrichtungen, wie Schikanen, Lochungen o. dgl. versehen sein. Auch kann der Einstromspalt 7 konisch ausgeführt sein, um so eine Vergleichmäßigung des anströmenden $CO_2$ zu erreichen u. dgl. mehr.

**Patentansprüche**

1. Verfahren zur Extraktion von Inhaltsstoffen aus Naturprodukten mittels eines unter Druck stehenden Fluids, insbesondere zur Entkoffeinierung von Teeblättern mittels $CO_2$, wobei anschließend die Inhaltsstoffe an einen Adsorbenten, vorzugsweise Aktivkohle, gebunden werden,
dadurch gekennzeichnet,
daß eine Schichtdicke des Naturproduktes vorgesehen wird, die klein im Verhältnis zur Schichtabmessung quer zur Strömungsrichtung ist und das Fluid durch die Schicht des Naturproduktes mit sich in Strömungsrichtung ändernder, insbesondere steigender Geschwindigkeit geführt wird.

2. Verfahren nach Anspruch 1,
dadurch gekennzeichnet,
daß das beladene Fluid ohne Zeitverzug unmittelbar nach Verlassen der Naturproduktschicht dem Adsorbenten zugeführt wird.

3. Verfahren nach Anspruch 1 oder 2,
dadurch gekennzeichnet,
daß das Fluid beim Anströmen der Schicht des Naturproduktes eine große Durchströmfläche und bei Verlassen der Naturproduktschicht eine demgegenüber wesentlich verkleinerte Abströmfläche durchströmt.

4. Verfahren nach einem der vorausgehenden Ansprüche,
dadurch gekennzeichnet,
daß das Fluid in wechselnder Folge eine Naturproduktschicht, den Adsorbenten, erneut eine Naturproduktschicht, wieder einen Adsorbenten und so fort durchströmt.

5. Vorrichtung zur Durchführung des Verfahrens nach Anspruch 1 oder einem der folgenden,
dadurch gekennzeichnet,
daß sie als im wesentlichen zylindrischer Hochdruckbehälter (1) mit im wesentlichen kreiszylinderförmigen Einsätzen (5 bzw. 6) zur Aufnahme des Naturproduktes und/oder des Adsorbenten ausgebildet und die Fluidführung von einem äußeren Bereich (7) des Zylinders in das Innere (12) des Zylinders vorgesehen ist.

6. Vorrichtung nach Anspruch 5,
dadurch gekennzeichnet,
daß der zylindrische Hochdruckbehälter (1) mit einem gasdichten Boden (3) und gasdichtem Deckel (4) ausgerüstet ist und der äußere Einsatz zur Aufnahme des Naturproduktes einen geringfügigen Abstand zur Innenfläche der zylindrischen Außenwand zur Bildung einer zylindrischen Anströmfläche für das Fluid aufweist, wobei der Fluidabzug nach Durchströmen des Naturproduktes sowie eines Adsorbenten am zentrischen zylindrischen Innenraum der Vorrichtung vorgesehen ist.

7. Vorrichtung nach einem der vorangehenden Ansprüche,
dadurch gekennzeichnet,
daß eine Mehrzahl von Einsätzen (5, 6) zur Aufnahme des Naturproduktes und des Adsorbenten konzentrisch zueinander in der Vorrichtung vorgesehen ist.

8. Vorrichtung nach Anspruch 7,
dadurch gekennzeichnet,
daß die Folge von zylindrischen Einsatzen (5, 6) mit Naturprodukten und Adsorbenten abwechselnd vorgesehen ist.

9. Vorrichtung nach einem der vorangehenden Ansprüche,
dadurch gekennzeichnet,
daß die jeweils außen liegende Anströmwand (8) für das Fluid mit Einrichtungen, wie Schikanen, Lochungen o. dgl. zur gleichmäßigen Verteilung des das Naturprodukt anströmenden Fluids ausgerüstet ist.

10. Vorrichtung nach Anspruch 5,
dadurch gekennzeichnet,
daß der Adsorbent (6'') in einem inneren Einsatzkorb und das Naturproduut im Ringraum (14) zwischen dem Adsorbenten (6'') und der Behälterwand (2'') eingebracht ist, wobei die Behälterwand (2'') wenigsten bereichsweise porös und von dem Fluid durchströmbar ausgebildet ist.

## Claims

1. A process for the extraction of constituent substances from natural products by means of a pressurised fluid, in particular for the decaffination of tealeaves by means of $CO_2$, wherein the constituent substances are then bound to an adsorbent, preferably activated carbon, characterised in that the natural product is provided in a layer of a thickness which is small in relation to the dimension to the layer transversely with respect to the direction of flow and the fluid is passed through the layer of the natural product at a rate which changes and in particular increases in the direction of flow.

2. A process according to claim 1 characterised in that the charged fluid is passed to the adsorbent without delay directly after leaving the layer of natural product.

3. A process according to claim 1 or claim 2 characterised in that, when the fluid flows to the layer of the natural product, the fluid flows through a large through-flow area and, when it leaves the layer of the natural product, it flows through a discharge flow area which is substantially reduced in comparison therewith.

4. A process according to one of the preceding claims characterised in that the fluid flows in alternate sequence through a layer of natural product, the absorbent, again a layer of natural product, again an adsorbent, and so forth.

5. Apparatus for carrying out the process according to claim 1 or one of the following claims characterised in that it is in the form of a substantially cylindrical high-pressure container (1) with substantially circular-cylindrical inserts (5 and 6 respectively) for accommodating the natural product and/or the adsorbent, and the fluid passes from an outer region (7) of the cylinder into the interior (12) of the cylinder.

6. Apparatus according to claim 5 characterised in that the cylindrical high-pressure container (1) is provided with a gas-tight bottom (3) and a gas-tight cover (4) and the outer insert for accommodating the natural product is at a small spacing from the inside surface of the cylindrical outside wall to form a cylindrical feed flow area for the fluid, wherein the removal of fluid occurs at the central cylindrical inside space of the apparatus after the fluid has flowed through the natural product and an adsorbent.

7. Apparatus according to one of the preceding claims characterised in that a plurality of inserts (5, 6) for accommodating the natural product and the adsorbent is arranged in concentric relationship in the apparatus.

8. Apparatus according to claim 7 characterised in that the sequence of cylindrical inserts (5, 6) with natural products and adsorbents is an alternate one.

9. Apparatus according to one of the preceding claims characterised in that the respective outwardly disposed feed flow wall (8) for the fluid is provided with means such as chicanes, perforations or the like for uniform distribution of the fluid which is flowing to the natural product.

10. Apparatus according to claim 5 characterised in that the adsorbent (6'') is disposed in an inner insert basket and the natural product is disposed in the annular space (14) between the adsorbent (6'') and the container wall (2''), wherein the container wall (2'') is porous at least in a region-wise manner and is designed to permit the fluid to flow therethrough.

## Revendications

1. Procédé pour l'extraction de substances contenues dans des produits naturels au moyen d'un fluide sous pression, en particulier pour la décaféination de feuilles de thé au moyen de $CO_2$, les substances extraites étant ensuite fixées sur un adsorbant, de préférence du charbon actif, caractérisé en ce qu'il est prévu une épaisseur de couche du produit naturel qui est petite par rapport à la dimension de la couche transversalement à la direction d'écoulement et en ce que le fluide est amené à passer à travers la couche du produit naturel avec une vitesse qui varie, notamment augmente, dans le sens d'écoulement.

2. Procédé selon la revendication 1, caractérisé en ce que le fluide chargé est amené sans délai directement à l'adsorbant après avoir quitté la couche de produit naturel.

3. Procédé selon la revendication 1 ou 2, caractérisé en ce que le fluide passe, en affluant à la couche du produit naturel, par une grande aire de traversée et, en quittant la couche de produit naturel, par une aire d'écoulement côté aval sensiblement réduite par rapport à l'aire de traversée.

4. Procédé selon l'une quelconque des revendications précédentes, caractérisé en ce que le fluide traverse successivement de manière alternée une couche de produit naturel, l'adsorbant, à nouveau une couche de produit naturel, puis à nouveau un adsorbant et ainsi de suite.

5. Dispositif pour la mise en oeuvre du procédé selon l'une quelconque des revendications précédentes, caractérisé en ce qu'il est réalisé en tant que récipient sensiblement cylindrique (1) sous haute pression avec des garnitures sensiblement cylindriques circulaires (5; 6) pour recevoir respectivement le produit naturel et/ou l'adsorbant et en ce que le guidage du fluide est prévu à partir d'une région extérieure (7) du cylindre vers l'intérieur (12) du cylindre.

6. Dispositif selon la revendication 5, caractérisé en ce que le récipient cylindrique (1) sous haute pression est équipé d'un fond (3) étanche aux gaz et d'un couvercle (4) étanche aux gaz et en ce que la garniture extérieure destinée à recevoir le produit naturel se trouve à une faible distance de la surface intérieure de la paroi cylindrique extérieure pour former une aire d'afflux cylindrique pour le fluide, le soutirage du fluide, après son écoulement à travers le produit naturel et un adsorbant, étant prévu au niveau de l'espace intérieur cylindrique central du dispositif.

7. Dispositif selon l'une quelconque des revendications précédentes, caractérisé en ce qu'il est prévu plusieurs garnitures (5, 6) destinées à recevoir respectivement le produit naturel et l'adsorbant et disposées concentriquement les unes par rapport aux autres dans le dispositif.

8. Dispositif selon la revendication 7, caractérisé en ce que la succession de garnitures cylindriques (5, 6) à produits naturels et adsorbants est prévue de façon qu'elles alternent.

9. Dispositif selon l'une quelconque des revendications précédentes, caractérisé en ce que la paroi d'afflux (8), située chaque fois à l'extérieur, pour le fluide est munie de déflecteurs tels que chicanes, trous ou analogues en vue de la répartition uniforme du fluide affluant au produit naturel.

10. Dispositif selon la revendication 5, caractérisé en ce que l'adsorbant (6'') est placé dans un panier intérieur à mettre en place et le produit naturel dans un espace annulaire (14) entre l'adsorbant (6'') et la paroi (2'') du récipient, la paroi (2'') du récipient étant réalisée au moins par endroits de façon à être poreuse et susceptible d'être traversée par le fluide.

FIG. 1

FIG. 2

CO2

4"

11"

CO2

10"

poröses
Hemd

8"

9"

14

A.-
Kohle

A.-
Kohle

1"

6"

Kaffeebohnen

2"

12"

3"

FIG. 3